# EUROPEAN PATENT SPECIFICATION

(11) **EP 2 309 987 B1**
(45) Date of publication and mention of the grant of the patent: **29.08.2012**
(21) Application number: 09774527.7
(22) Date of filing: 02.07.2009
(51) Int. Cl.: A61K 9/16, A61K 9/20, A61K 31/4709

(54) **MELT GRANULATION PROCESS**
SCHMELZGRANULATIONSVERFAHREN
PROCESSUS DE GRANULATION PAR FUSION

(30) Priority: 03.07.2008 US 133851
(43) Date of publication of application: 20.04.2011
(73) Proprietor: Novartis AG, 4056 Basel (CH)
(72) Inventor: QIU, Zhihui, Bridgewater NJ 08807 (US); LI, Shoufeng, Basking Ridge NJ 07920 (US); BENJAMIN, Daniel, Eliot, Highland Park NJ 08904 (US)
(74) Representative: Roth, Peter Richard
(86) International application number: PCT/US2009/049548
(87) International publication number: WO 2010/003078

(56) References cited:
- WO-A2-2007/064719
- US-A1- 2001 036 476

## Description

### Field of the Invention

The present invention relates to a process for making solid oral dosage forms in which the therapeutic compound is a quinoline compound. The process features the use of melt granulation with an extruder. Such solid oral dosage forms are useful for the treatment and prevention of proliferative diseases including cancer.

### Background of the Invention

### Summary of the Invention

The present invention relates to the pharmaceutical granulation process that can convert unwanted polymorph or mixture of different physical forms of active pharmaceutical ingredient to the desirable form and ensure only the desirable form is present in the drug product. For 4-amino-5-fluoro-3-[5-(4-methylpiperazin-1-yl)-1H-benzimidazol-2-yl]quinolin-2(1H)-one lactic acid salt, the manufacturing of the clinical desirable anhydrous form tends to have monohydrate form as impurity, which is less soluble. However, the monohydrate form can completely and irreversibly convert to anhydrous form at temperature above 140°C. In current invention, melt granulation is used to provide the high temperature to produce granules of pure anhydrous form regardless the composition of starting material is monohydrate or mixture of monohydrate and anhydrate forms.

### Detailed Description of the Invention

The present invention relates to a process for preparing pharmaceutical compositions, especially solid oral dosage forms, of a quinolide compound, in particular, 4-amino-5-fluoro-3-15-(4-methylpiperazin-1-yl)-1H-benzimidazol-2-yl]quinolin-2(1H)-one lactic acid salt. The inventive process features melt granulation, using an extruder.

As used herein, the term "pharmaceutical composition" means a mixture containing a therapeutic compound to be administered to a mammal, e.g., a human in order to prevent, treat or control a particular disease or condition affecting the mammal.

As used herein, the term "pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

"Therapeutic compound", as used hereinis 4-amino-5-fluoro-3-[5-(4-mothylpiperazin-1-yl)-1H-benzimidazol-2-yl]-quinolin-2(1H)-one and has the formula (I):

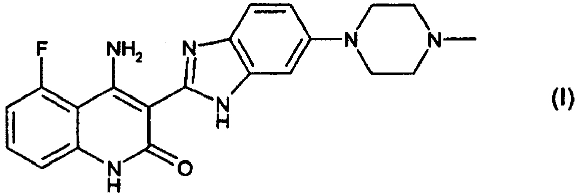

The therapeutic compound is the tactic acid salt form of the compound of formula (I) which is 4-amino-5-fluoro-3-[5-(4-methylpiperazin-1-yl)-1H-benzimidazol-2-yl]quinolin-2(1H)-one lactic acid salt.

WO 2006/127926 provides information of polymorph and solvate forms of 4-amino-5-fluoro-3-[5-(4-methylpiperazln-1-yl)-1H-benzimidazol-2-yl]quinolin-2(1H)-one.

WO 2007/064719 discloses a process for making granules comprising 4-amino-5-fluoro-3-[5-(4-methylpiperazin-1-yl)-1H-benzimidazol-2-yl]quinolin-2(1H)-one lactic acid salt by wet granulation.

As used herein, the term "granulation excipient" refers to any pharmaceutically acceptable material or substance that can be melt granulated with therapeutic compound as further described below. The granulation excipient, e.g., can be a polymer or a non-polymeric material.

As used herein, the term "polymer" refers to a polymer or mixture of polymers that have a glass transition temperature, softening temperature or melting temperature by itself or in combination not exceeding the melting point (or melting range) of the therapeutic compound. The glass transition temperature ("Tg") is the temperature at which such polymer's characteristics change from that of highly viscous to that of relatively less viscous mass. Types of polymers include, but are not limited to, water-soluble, water-swellable, water insoluble polymers and combinations of the foregoing.

Examples of polymers include, but are not limited to:
homopolymers and copolymers of N-vinyl lactams, e.g., homopolymers and copolymers of N-vinyl pyrrolidone (e.g., polyvinylpyrrolidone), copolymers of N-vinyl pyrrolidone and vinyl acetate or vinyl propionate;
cellulose esters and cellulose ethers (e.g., methylcellulose and ethylcellulose) hydroxyalkylcelluloses (e.g., hydroxypropylcellulose), hydroxyalkylalkylcelluloses (e.g., hydroxypropylmethylcellulose), cellulose phthalates (e.g., cellulose acetate phthalate and hydroxylpropylmethylcellulose phthalate) and cellulose succinates (e.g., hydroxypropylmethylcellulose succinate or hydroxypropylmethylcellulose acetate succinate);
high molecular polyalkylene oxides such as polyethylene oxide and polypropylene oxide and copolymers of ethylene oxide and propylene oxide;
polyacrylates and polymethacrylates (e.g., methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), poly(hydroxyalkyl methacrylates));
polyacrylamides;
vinyl acetate polymers such as copolymers of vinyl acetate and crotonic acid, partially hydrolyzed polyvinyl acetate;
polyvinyl alcohol; and
oligo- and polysaccharides, such as carrageenans, galactomannans and xanthan gum, or mixtures of one or more thereof.

As used herein, the term "plasticizer" refers to a material that may be incorporated into the pharmaceutical composition in order to decrease the glass transition temperature and the melt viscosity of a polymer by increasing the free volume between polymer chains. Plasticizers, for example, include, but are not limited to, water; sorbitol; citrate esters (e.g., triethylcitrate, triacetin); low molecular weight poly(alkylene oxides) (e.g., poly(ethylene glycols), poly(propylene glycols), poly(ethylenelpropylene glycols)); glycerol, pentaerythritol, glycerol monoacetate, diacetate or triacetate; propylene glycol; sodium diethyl sulfosuccinate; and the therapeutic compound itself. The plasticizer can be present in concentration from about 0-15%, e.g., 0.5-5% by weight of the pharmaceutical composition. Examples of plasticizers can also be found in The Handbook of Pharmaceutical Additives, Ash et al., Gower Publishing (2000).

Non-polymeric granulation excipients include, but are not limited to, esters, hydrogenated oils, oils, natural waxes, synthetic waxes, hydrocarbons, fatty alcohols, fatty acids, monoglycerides, diglycerides, triglycerides and mixtures thereof.

Examples of esters, such as glyceryl esters include, but are not limited to, glyceryl monostearate, e.g., CAPMUL GMS from Abitec Corp. (Columbus, OH); glyceryl palmitostearate; acetylated glycerol monostearate; sorbitan monostearate, e.g., ARLACEL 60 from Uniqema (New Castle, DE); and cetyl palmitate, e.g., CUTINA CP from Cognis Corp. (Düsseldorf, Germany), magnesium stearate and calcium stearate.

Examples of hydrogenated oils include, but are not limited to, hydrogenated castor oil; hydrogenated cottonseed oil; hydrogenated soybean oil; and hydrogenated palm oil. An example of oil include sesame oil.

Examples of waxes include, but are not limited to, carnauba wax, beeswax and spermaceti wax. Examples of hydrocarbons include, but are not limited to, microcrystalline wax and paraffin. Examples of fatty alcohols, i.e., higher molecular weight nonvolatile alcohols that have from about 14 to about 31 carbon atoms include, but are not limited to, cetyl alcohol, e.g., CRODACOL C-70 from Croda Corp. (Edison, NJ) ; stearyl alcohol, e.g., CRODACOL S-95 from Croda Corp; lauryl alcohol; and myristyl alcohol. Examples of fatty acids which may have from about 10 to about 22 carbon atoms include, but are not limited to, stearic acid, e.g., HYSTRENE 5016 from Crompton Corp. (Middlebury, CT); decanoic acid; palmitic acid; lauric acid; and myristic acid.

As used herein, the term "melt granulation" refers to the following compounding process that comprises the steps of:
(a) forming a mixture of the therapeutic compound with at least one granulation excipient;
(b) granulating the mixture using an extruder while heating the mixture to a temperature that is less than the melting point (or melting range) of the therapeutic compound; and
(c) cooling the extrudate to room temperature, for example, at a controlled rate.

The heating and mixing of the therapeutic compound and the granulation excipient to form an internal phase of granules (i.e., from the extrudate) is accomplished by the use of an extruder. The granulation excipient, e.g., can be present in an amount from about 1% to about 50% by weight of the composition. In one embodiment, the granulation excipient may be present in an amount from about 3% to about 25% by weight of the composition. Unlike granules made during a wet granulation process, the melt granulation process of the present invention does not necessarily require a granulation fluid, e.g., water, methanol, ethanol, isopropanol or acetone during the granulation process.

The resulting granules are, e.g., particles of the therapeutic compound coated or substantially coated by the granulation excipient, or alternatively, particles of the therapeutic compound embedded or substantially embedded with or within the granulation excipient.

In general, an extruder includes a rotating screw(s) within a stationary barrel with an optional die located at one end of the barrel. Along the entire length of the screw, distributive kneading of the materials (e.g., the therapeutic compound, release retarding material, and any other needed excipients) is provided by the rotation of the screw(s) within the barrel. Conceptually, the extruder can be divided into at least three sections: a feeding section; a heating section and a metering section. In the feeding section, the raw materials are fed into the extruder, e.g., from a hopper. In the heating section, the raw materials are heated to a temperature less than the melting temperature of the therapeutic compound. After the heating section is a metering section in which the mixed materials are extruded through an optional die into a particular shape, e.g., granules or noodles. Types of extruders particularly useful in the present invention are single-, twin- and mufti-screw extruders, optionally configured with kneading paddles.

Once the granules are obtained, the granules may be formulated into oral forms, e.g., solid oral dosage forms, such as tablets, pills, lozenges, caplets, capsules or sachets, by adding additional conventional excipients which comprise an external phase of the pharmaceutical composition. The external phase of the pharmaceutical composition can also comprise an additional therapeutic compound. Such solid oral dosage forms, e.g., are unit oral dosage forms. Examples of such excipients include, but are not limited to, release retardants, plasticizers, disintegrants, binders, lubricants, glidants, stabilizers, fillers and diluents. One of ordinary skill in the art may select one or more of the aforementioned excipients with respect to the particular desired properties of the solid oral dosage form by routine experimentation and without any undue burden. The amount of each excipient used may vary within ranges conventional in the art. The following references disclose techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003).

As used herein, the term "release retardant" refers to any material or substance that slows the release of a therapeutic compound from a pharmaceutical composition when orally ingested. Various sustained release systems, as known In the art, can be accomplished by the use of a release retarding component, e.g., a diffusion system, a dissolution system and/or an osmotic system. A release retardant can be polymeric or non-polymeric in nature. The pharmaceutical compositions of the present invention can include, e.g., at least five percent of a release retardant by weight of the composition if a sustained release composition is desired.

Examples of pharmaceutically acceptable disintegrants include, but are not limited to, starches; celluloses; sodium starch glycolate, cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; and guar gum. The disintegrant may be present In an amount from about 0% to about 10% by weight of the composition. In one embodiment, the disintegrant is present in an amount from about 0.1% to about 8% by weight of composition.

Examples of pharmaceutically acceptable binders include, but are not limited to, starches; celluloses and derivatives thereof, for example, microcrystalline cellulose, e.g., AVICEL PH from FMC (Philadelphia, PA), povidone, coployvidone, hydroxypropyl cellulose, hydroxylethyl cellulose and hydroxylpropylmethyl cellulose METHOCEL from Dow Chemical Corp. (Midland, Ml); and gelatin. The binder may be present in an amount from about 0% to about 50%, e.g., 10-40% by weight of the composition.

Examples of pharmaceutically acceptable lubricants and pharmaceutically acceptable glidants include, but are not limited to, starches, talc, tribasic calcium phosphate, magnesium stearate, calcium stearate, stearic acid, sodium stearyl fumarate, hydrogenated oil, compritol, polyethylene glycol. The lubricant may be present in an amount from about 0% to about 10% by weight of the composition. In one embodiment, the lubricant may be present in an amount from about 0.1 % to about 1.5% by weight of composition. The glidant may be present in an amount from about 0.1 % to about 10% by weight.

Examples of pharmaceutically acceptable fillers and pharmaceutically acceptable diluents include, but are not limited to, compressible sugar, dextrates, dextrin, dextrose, lactose, mannitol, microcrystalline cellulose, powdered cellulose, sorbitol, sucrose and talc. The filter and/or diluent, e.g., may be present in an amount from about 15% to about 40% by weight of the composition.

To make pharmaceutical compositions of the present invention the therapeutic compound and a granulation excipient are blended in a ratio in a range of 99:1 to 1:1 (on a dry weight basis) prior to, or upon addition into the hopper of an extruder. In one exemplary embodiment, this ratio between the therapeutic compound and granulation excipient can be in a range of 97:3 to 40:60 (on a dry weight basis). Yet in another alternative embodiment, the ratio can be in a range of 97:3 to 75:25 (on a dry weight basis). Optionally, a plasticizer can be added to the internal phase.

The mixture is heated to a temperature(s) less than the melting temperature of the therapeutic compound. The heating temperature is preferably below 140°C. As the mixture is being heated, it is also being kneaded by the screw(s) of the extruder. The mixture is maintained at the elevated temperature and blended for a time sufficient to form a granulated product. After the mixture is conveyed down the entire length of the barrel, a granulated product (being the extrudate) is obtained, and the granulated mixture is cooled.

After cooling, the extrudate can be milled and subsequently screened through a sieve. The granules (which constitute the internal phase of the pharmaceutical composition) are then combined with solid oral dosage form excipients (the external phase of the pharmaceutical composition), i.e., fillers, binders, disintegrants, lubricants and etc. The combined mixture may be further blended, e.g., through a V-blender, and subsequently compressed or molded into a tablet, for example a monolithic tablet, or encapsulated by a capsule.

Once the tablets are obtained, they can be optionally coated with a functional or nonfunctional coating as known in the art. Examples of coating techniques include, but are not limited to, sugar coating, film coating, microencapsulation and compression coating. Types of coatings include, but are not limited to, enteric coatings, sustained release coatings, controlled-release coatings.

The utility of all the pharmaceutical compositions of the present invention may be observed in standard clinical tests in, for example, known indications of drug dosages giving therapeutically effective blood levels of the therapeutic compound; for example using dosages in the range of 2.5-1000 mg of therapeutic compound per day for a 75 kg mammal, e.g., adult and in standard animal models.

The present invention provides a method of treatment of a subject suffering from a disease, condition or disorder treatable with the therapeutic compound comprising administering a therapeutically effective amount of the pharmaceutical composition of the present invention to a subject in need of such treatment.

The following examples are illustrative, but do not serve to limit the scope of the invention described herein. The examples are meant only to suggest a method of practicing the present invention.

### Example 1

| | **Ingredient** | **Percentage (w/w)** | **Amount per tablet (mg)** |
|---|---|---|---|
| Internal phase | | | |
| | Monohydrate 4-amino-5-fluoro-3-[5-(4-methylpiperazin-1- yl)-1H-benzimidazol-2-yl]quinolin-2(1H)-one lactic acid salt | 40.00% | 260.0 |
| | Hydroxylpropyl cellulose EXF | 4.00% | 26.0 |
| External phase | | | |
| | Microcrystalline cellulose (AVICEL PH102) | 50.5% | 328.2 |
| | Croscarmellose sodium | 5.00% | 32.5 |
| | Magnesium stearate | 0.50% | 3.3 |
| Total | | 100% | 650.0 |

The internal phase ingredients, i.e., monohydrate 4-amino-5-fluoro-3-[5-(4-methylpiperazin-1-yl)-1H-benzimidazol-2-yl]quinolin-2(1H)-one lactic acid salt, and hydroxypropyl cellulose available as Klucel EXF from Aqualon are combined and blended in a bin blender for about 200 rotations. The blend is introduced into the feed section, or hopper, of a twin screw extruder. A suitable twin screw extruder is the PRISM 16 mm pharmaceutical twin screw extruder available from Thermo Electron Corp. (Waltham, Massachusetts).

Located at the end of the twin screw extruder is a die with a bore of approximately 3 mm. The twin screw extruder is configured with five individual barrel zones, or sections, that can independently adjusted to different parameters. Starting from the hopper to the die, the zones are respectively heated to the following temperatures: 145°C, 145°C, 120°C, 80°C and 40°C. The screw speed is set to 150 rpm, but can be as high as 400 rpm.

The extrudate, or granules, from the extruder are then cooled to room temperature by allowing them to stand from approximately 15-20 minutes. The cooled granules, are subsequently sieved through an 18 mesh screen (i.e., a one mm screen). The cooled granules comprise anhydrous 4-amino-5-fluoro-3-[5-(4-methylpiperazin-1-yl)-1H-benzimidazol-2-yl]quinolin-2(1H)-one lactic acid salt.

For the external phase, Avicel and croscarmellose sodium were blended with the obtained granules using a suitable bin blender for approximately 200 rotations. The magnesium stearate is first passed through an 30 mesh. The magnesium stearate is then blended with the mixture for approximately 60 rotations. The resulting final blend is compressed into tablets using a conventional rotary tablet press (Manesty Beta Press) using a compression force ranging between 6 kN and 40 kN, or Claver press suing compression force ranging from 5-15 kN. The resulting tablets are monolithic and having a hardness ranging from 100-400 N. Tablets having hardness ranging from 200-400 N resulted in acceptable friability of less than 1.0% w/w after 500 drops.

Similar process was used to prepare the following tablets.

### Example 2

| | **Ingredient** | **Percentage *(w*/*w)*** | **Amount per tablet (mg)** |
|---|---|---|---|
| Internal phase | | | |
| | Monohydrate 4-amino-5-fluoro-3-[5-(4-methylpiperazin-1-yl)-1H-benzimidazol-2-yl]quinolin-2(1H)-one lactic acid salt | 90.00% | 765.0 |
| | Hydroxypropyl cellulose (HPC-EXF) | 10.0% | 85.0 |
| External phase | | | |
| | **Magnesium stearate** | Trace (external lubrication) | Trace |
| Total | | 100% | 850 |

| | **Force (kN)** | **Hardness (N)** | **Friability (%)** | **Disintegration Time (min)** |
|---|---|---|---|---|
| **Example 1** | 7 | 300 | 0.4 | ~2.5 |
| **Example 2** | 12 | 200 | 0.3 | -30 |

## Claims

1. A process for making a pharmaceutical composition comprising the steps of:
(a) combining the therapeutic compound 4-amino-5-fluoro-3-[5-(4-methylpiperazin-1-yl)-1H-benzimidazol-2-yl]quinolin-2(1H)-one lactic acid salt with at least one granulation excipient to form a mixture;
(b) kneading said mixture in an extruder while heating said mixture to a heating temperature less than a melting point of said therapeutic compound; and
(c) extruding said mixture to form granules.

2. The process according to Claim 1 where said compound is monohydrate 4-amino-5-fluoro-3-[5-(4-methylpiperazin-1-yl)-1H-benzimidazol-2-yl]quinolin-2(1H)-one lactic acid salt.

3. The process according to Claim 1 or 2, where said granulation excipient is selected from the group consisting of:
homopolymers and copolymers of N-vinyl pyrrolidone, copolymers of N-vinyl pyrrolidone and vinyl acetate or vinyl propionate;
methylcellulose, ethylcellulose, hydroxypropylcellulose, hydroxypropylmethylcellulose, cellulose acetate phthalate, hydroxylpropylmethylcellulose phthalate, hydroxypropylmethylcellulose succinate , hydroxypropylmethylcellulose acetate succinate;
polyethylene oxide, polypropylene oxide, copolymers of ethylene oxide and propylene oxide;
methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), poly(hydroxyalkyl methacrylates);
polyacrylamides;
copolymers of vinyl acetate and crotonic acid, partially hydrolyzed polyvinyl acetate;
polyvinyl alcohol;
carrageenans, galactomannans and xanthan gum, or mixtures of one or more thereof;
water; sorbitol;
triethylcitrate, triacetin; poly(ethylene glycols, poly(propylene glycols), poly(ethylene/propylene glycols);
glycerol, pentaerythritol, glycerol monoacetate, diacetate or triacetate; propylene glycol; sodium diethyl sulfosuccinate;
glyceryl monostearate,; glyceryl palmitostearate; acetylated glycerol monostearate; sorbitan monostearate,; cetyl palmitate, magnesium stearate, calcium stearate;
hydrogenated castor oil; hydrogenated cottonseed oil; hydrogenated soybean oil; hydrogenated palm oil, sesame oil;
carnauba wax, beeswax, spermaceti wax, microcrystalline wax, paraffin;
cetyl alcohol; stearyl alcohol; lauryl alcohol; myristyl alcohol, stearic acid; decanoic acid; palmitic acid; lauric acid; and myristic acid.

4. The process according to Claim 1 or 2, where said granulation excipient is selected from the group consisting of sorbitol, hydroxypropylcellulose and propylene ethylene glycol.

5. The process according to any preceding Claim, where the heating temperature below140°C.

6. The process according to any preceding Claim, where said granules comprises anhydrous 4-amino-5-fluoro-3-[5-(4-methylpiperazin-1-yl)-1H-benzimidazol-2-yl]quinolin-2(1H)-one lactic acid salt.

## Patentansprüche

1. Verfahren zur Herstellung einer pharmazeutischen Zusammensetzung, das die folgenden Stufen umfasst:
(a) Kombinieren der therapeutischen Verbindung 4-Amino-5-fluor-3-[5-(4-methylpiperazin-1-yl)-1H-benzimidazol-2-yl]chinolin-2(1H)-on-Milchsäuresalz mit mindestens einem Granulationshilfsstoff zur Bildung eines Gemisches;
(b) Kneten des Gemisches in einem Extruder unter Erwärmen des Gemisches auf eine Erwärmungstemperatur, die geringer ist als der Schmelzpunkt der therapeutischen Verbindung; und
(c) Extrudieren des Gemisches zur Bildung von Granalien.

2. Verfahren nach Anspruch 1, wobei die Verbindung 4-Amino-5-fluor-3-[5-(4-methylpiperazin-1-yl)-1H-benzimidazol-2-yl]chinolin-2(1H)-on-Milchsäuresalz-monohydrat ist.

3. Verfahren nach Anspruch 1 oder 2, worin der Granulationshilfsstoff aus der Gruppe ausgewählt ist, die aus:
Homopolymeren und Copolymeren von N-Vinylpyrrolidon, Copolymeren von N-Vinylpyrrolidon und Vinylacetat oder Vinylpropionat;
Methylcellulose, Ethylcellulose, Hydroxypropylcellulose, Hydroxypropylmethylcellulose, Celluloseacetat-phthalat, Hydroxypropylmethylcellulose-phthalat, Hydroxypropylmethylcellulose-succinat, Hydroxypropylmethylcelluloseacetat-succinat;
Polyethylenoxid, Polypropylenoxid, Copolymeren von Ethylenoxid und Propylenoxid;
Methacrylsäure/Ethylacrylat-Copolymeren, Methacrylsäure/Methylmethacrylat-Copolymeren, Butytmethacrylatl2-Dimethylaminoethyl-methacrylat-Copolymeren, Poly(hydroxyalkyl-acrylaten), Poly(hydroxyalkyl-methacrylaten);
Polyacrylamiden;
Copolymeren von Vinylacetat und Crotonsäure, partiell hydrolysiertem Polyvinylacetat;
Polyvinylalkohol;
Carrageenen, Galaktomannanen, Xanthangummi oder Gemischen von einem oder mehreren hiervon;
Wasser, Sorbitol;
Triethylcitrat, Triacetin; Poly(ethylenglykolen), Poly(propylenglykolen), Poly(ethylen/propylenglycolen);
Glycerin, Pentaerythrit, Glycerinmonoacetat, -diacetat oder -triacetat, Propylenglycol, Natriumdiethylsulfosuccinat;
Glycerylmonostearat, Glyceryl-palmitostearat, acetyliertem Glycerinmonostearat, Sorbitanmonostearat, Cetylpalmitat, Magnesiumstearat, Calciumstearat;
hydriertem Rizinusöl, hydriertem Baumwollkernöl, hydriertem Sojaöl, hydriertem Palmöl, Sesamöl;
Carnaubawachs, Bienenwachs, Walratwachs, mikrokristallinem Wachs, Paraffin;
Cetylalkohol, Stearylalkohol, Laurylalkohol, Myristylalkohol, Stearinsäure, Decansäure, Palmitinsäure, Laurinsäure und Myristinsäure besteht.

4. Verfahren nach Anspruch 1 oder 2, wobei der Granulationshilfsstoff aus der Gruppe ausgewählt ist, die aus Sorbitol, Hydroxypropylcellulose und Propylenethylenglykol besteht.

5. Verfahren nach einem der vorhergehenden Ansprüche, wobei die Erwärmungstemperatur unter 140 °C liegt.

6. Verfahren nach einem der vorhergehenden Ansprüche, wobei die Granalien wasserfreies 4-Amino-5-fluor-3-[5-(4-methylpiperazin-1-yl)-1H-benzimidazol-2-yl]chinolin-2(1H)-on-Milchsäuresalz umfassen.

## Revendications

1. Procédé de fabrication d'une composition pharmaceutique comprenant les étapes qui consistent à :
(a) combiner le composé thérapeutique sel d'acide lactique de 4-amino-5-fluoro-3-[5-(4-méthylpipérazin-1-yl)-1H-benzimidazol-2-yl]quinoléin-2(1H)-one avec au moins un excipient de granulation pour former un mélange ;
(b) malaxer ledit mélange dans une extrudeuse tout en chauffant ledit mélange à une température de chauffage inférieure à un point de fusion dudit composé thérapeutique ; et à
(c) extruder ledit mélange pour former des granulés.

2. Procédé selon la revendication 1, dans lequel ledit composé est le sel d'acide lactique de 4-amino-5-fluoro-3-[5-(4-méthylpipérazin-1-yl)-1H-benzimidazol-2-yl]quinoléin-2(1H)-one monohydrate.

3. Procédé selon la revendication 1 ou 2, dans lequel ledit excipient de granulation est choisi dans le groupe constitué par :
les homopolymères et copolymères de N-vinylpyrrolidone, les copolymères de N-vinylpyrrolidone et d'acétate de vinyle ou de propionate de vinyle ;
la méthylcellulose, l'éthylcellulose, l'hydroxypropylcellulose, l'hydroxypropylméthylcellulose, l'acétate phtalate de cellulose, le phtalate d'hydroxypropylméthylcellulose, le succinate d'hydroxypropylméthylcellulose, l'acétate succinate d'hydroxypropylméthylcellulose ;
l'oxyde de polyéthylène, l'oxyde de polypropylène, les copolymères d'oxyde d'éthylène et d'oxyde de propylène ;
les copolymères d'acide méthacrylique et d'acrylate d'éthyle, les copolymères d'acide méthacrylique et de méthacrylate de méthyle, les copolymères de méthacrylate de butyle et de méthacrylate de 2-diméthylaminoéthyle, les poly(acrylates d'hydroxyalkyle), les poly(méthacrylates d'hydroxyalkyle) ;
les polyacrylamides ;
les copolymères d'acétate de vinyle et d'acide crotonique, le polyacétate de vinyle partiellement hydrolyse ;
l'alcool polyvinylique ;
les carraghénines, les galacto-mannanes, la gomme xanthane, ou leurs combinaisons de deux, ou plus ;
l'eau ; le sorbitol ;
le citrate de triéthyle, la triacétine, les poly(éthylène glycols), les poly(propylène glycols), les poly(éthylène / propylène glycols) ;
le glycérol, le pentaérythritol, le monoacétate, le diacétate ou le triacétate de glycérol ; le propylène glycol ; le sulfosuccinate de diéthyle sodique ;
le monostéarate de glycéryle, le palmitostéarate de glycéryle, le monostéarate de glycérol acétylé ; le monostéarate de sorbitan, le palmitate de cétyle, le stéarate de magnésium et le stéarate de calcium ;
l'huile de ricin hydrogénée, l'huile de coton hydrogénée, l'huile de soja hydrogénée, l'huile de palme hydrogénée, l'huile de sésame ;
la cire de carnauba, la cire d'abeille, la cire de blanc de baleine, la cire microcristalline et la paraffine ;
l'alcool cétylique, l'alcool stéarylique, l'alcool laurylique, l'alcool myristylique, l'acide stéarique, l'acide décanoïque, l'acide palmitique, l'acide laurique, et l'acide myristique.

4. Procédé selon la revendication 1 ou 2, dans lequel ledit excipient de granulation est choisi dans le groupe constitué par le sorbitol, l'hydroxypropylcellulose et le propylène éthylène glycol.

5. Procédé selon l'une quelconque des revendications précédentes, dans lequel la température de chauffage est inférieure à 140 °C.

6. Procédé selon l'une quelconque des revendications précédentes, dans lequel lesdits granulés comprennent le sel d'acide lactique de 4-amino-5-fluoro-3-[5-(4-méthylpipérazin-1-yl)-1H-benzimidazol-2-yl]quinoléin-2(1H)-one anhydre.
